# EUROPEAN PATENT APPLICATION

(11) **EP 2 374 421 A2**
(43) Date of publication of application: **12.10.2011**
(21) Application number: 11250453.5
(22) Date of filing: 11.04.2011
(51) Int. Cl.: A61B 17/34

(54) **Hollow multi-lumen access port**

(30) Priority: 12.04.2010 US 323109 P; 18.02.2011 US 30164
(71) Applicant: Tyco Healthcare Group LP, New Haven, CT 06511 (US)
(72) Inventor: Kleyman, Gennady, Brooklyn, NY 11230 (US)
(74) Representative: Soames, Candida Jane

(57) **Abstract**

A surgical apparatus (10) for positioning within a tissue tract accessing an underlying body cavity includes a seal anchor member (100) that is adapted and configured to be placed within an incision (C) of a tissue (T) in a substantially sealed relation. The seal anchor member (100) includes a hollow body portion (4) and may also include a removable cap (2) including a plurality of lumens (8) that are each adapted and configured to receive a surgical instrument (I) therein.

## Description

### CROSS REFERENCE TO RELATED APPLICATION

The present application claims the benefit of and priority to U.S. Provisional Application Serial No. 61/323,109 filed on April 12, 2010, the entire contents of which are incorporated herein by reference.

### BACKGROUND

### Technical Field

The present disclosure relates to an access port for use in a surgical procedure. More particularly, the present disclosure relates to a seal anchor member adapted for insertion into an incision in tissue, and, for the sealed reception of one or more surgical objects such that a substantially fluid-tight seal is formed with both the tissue and the one or more surgical objects.

### Background of the Related Art

Today, many surgical procedures are performed through small incisions in the skin. Generally, such procedures are referred to as "endoscopic", unless performed on a patient's abdomen, in which case the procedures are referred to as "laparoscopic". Throughout the present disclosure, the term "minimally invasive" should be understood to encompass both endoscopic and laparoscopic procedures. As compared to the larger incisions typically required in traditional open surgical procedures, minimally invasive procedures result in reduced trauma and recovery time for the patient.

During a typical minimally invasive procedure, surgical objects, such as, surgical access devices (e.g. trocar and cannula assemblies) or endoscopes are inserted into the patient's body through an incision in the patient's tissue. In general, prior to the introduction the surgical object into the patient's body, insufflation gasses are used to enlarge the area surround the target surgical site to create a larger and more accessible work area. Accordingly, the maintenance of a substantially fluid-tight seal is desirable so as to prevent the escape of the insufflation gasses and the deflation or collapse of the enlarged surgical site.

To this end, various valves and seals have been developed for use during a minimally invasive procedure and are widely known in the art. However, a continuing need exits for a seal anchor member that can be inserted directly into the incision within the tissue and that can accommodate a variety of surgical objects while maintaining the integrity of the insufflated workspace.

### SUMMARY

The present disclosure relates to a surgical apparatus for positioning within a tissue tract accessing an underlying body cavity. The surgical apparatus includes a seal anchor member having a longitudinal axis and having a leading end and trailing end. The seal anchor member includes a body portion defining a lumen along a longitudinal axis of the seal anchor member. The seal anchor member also includes a cap that is releasably secured to the trailing end of the seal anchor member. The cap includes one or more openings extending therethrough and parallel to the longitudinal axis. Each opening of the cap may define a different diameter, however the diameters of the openings are less than the diameter of the lumen of the body portion. The body portion may also include a lip at the leading end of the seal anchor member to facilitate anchoring of the seal anchor member within the incision. In addition, the body portion may include a lip at the trailing end of the seal anchor member that releasably secures the cap to the body portion.

As discussed above, the body portion may include lips at the trailing and leading ends of the seal anchor member. Moreover, the body portion may include an intermediate portion disposed between the lips that has an arcuate configuration to facilitate insertion of the seal anchor member within the tissue tract.

The seal anchor member may be formed from a semi-rigid or from a compressible material. By way of example only, the seal anchor member may be formed from a rubber, polyurethane, or foam material. Moreover, to facilitate maintenance of the structural integrity of the body portion of the seal anchor member while inserted within the incision of the tissue, the body portion may include grooves or ribbing.

In an embodiment, the present invention relates to a surgical apparatus for positioning within a tissue tract accessing an underlying body cavity. The apparatus may include a seal anchor member having a longitudinal axis, the seal anchor member including a compressible body portion having an inner surface defining a lumen along a longitudinal axis of the seal anchor member, the lumen defining a first diameter and having ribbing along the inner surface. The ribbing may increase the structural rigidity of the flexible body portion. The ribbing may resist compression when placed within the incision. The ribbing may extend longitudinally along the inner surface. The ribbing may extend from the proximal end of the compressible body portion to the distal end of the compressible body portion. The surgical apparatus may also include a cap that is releasably securable to a proximal end of the seal anchor member. The cap may include one or more openings extending along the longitudinal axis, each opening defining a diameter, each diameter being less than the first diameter of the lumen of the seal anchor member. Each opening of the cap may be sized to receive a surgical object.

In an embodiment, the present invention relates to a surgical apparatus for positioning within a tissue tract accessing an underlying body cavity, which comprises a body portion having an inner surface defining a lumen along a longitudinal axis of the seal anchor member, the body portion being selectively compressible so as to be inserted within a tissue tract, the lumen having ribbing along the inner surface, the ribbing increasing the structural rigidity of the flexible body portion such that, after the body portion has already been positioned within the incision, the ribbing helps maintain the lumen open by resisting compression forces exerted on the body portion by the tissue tract. The ribbing may extend longitudinally along the inner surface. The ribbing may extend from the proximal end of the compressible body portion to the distal end of the compressible body portion.

These and other features of the apparatus disclosed herein will be explained in greater detail below with reference to the appended figures.

### BRIEF DESCRIPTION OF THE DRAWINGS

Various embodiments of the present disclosure are described hereinbelow with reference to the appended drawings, wherein:

Fig. 1 is a front perspective view of a surgical apparatus in accordance with the principles of the present disclosure;

Fig. 2 is a bottom perspective view of the surgical apparatus of Fig. 1;

Fig. 3 is a cross-sectional view of the surgical apparatus of Fig. 1 taken along section line 3-3;

Fig. 4 is a front perspective view of the surgical apparatus of Fig. 1 shown in a first state and placed within an incision of a tissue; and

Fig. 5 is a front perspective view of the surgical apparatus of Fig. 1 shown in a second state and placed within an incision of a tissue.

### DETAILED DESCRIPTION OF THE EMBODIMENTS

In the drawings and in the description which follows, in which like references numerals identify similar or identical elements, the term "proximal" or "trailing" will refer to the end of the apparatus which is closest to the clinician during use, while the term "distal" or "leading" will refer to the end which is farthest from the clinician, as is traditional and known in the art.

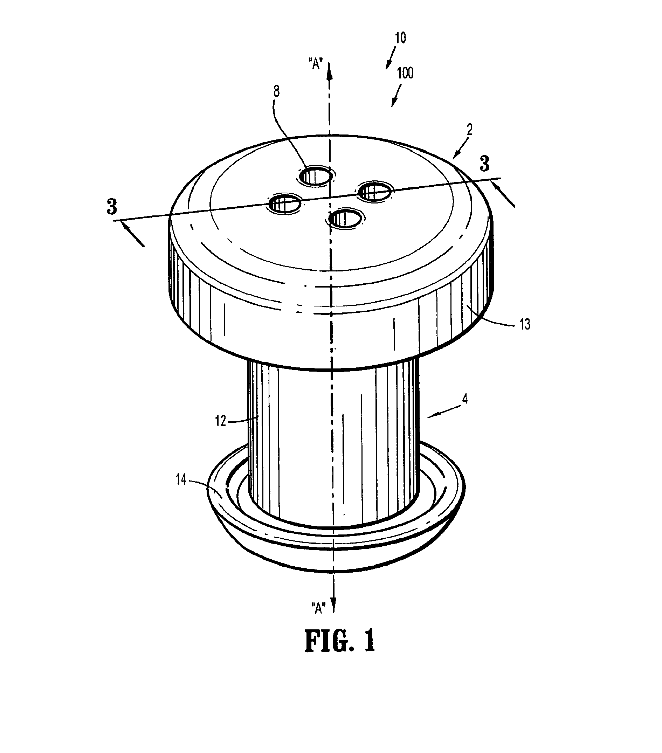

With reference to Figs. 1-5, a surgical apparatus 10 for use in a surgical procedure, e.g., a minimally invasive procedure, will now be described. Surgical apparatus 10 includes a seal anchor member 100 that is configured and adapted to be placed within an incision "C" of a tissue "T". The seal anchor member 100 is configured and adapted to maintain the incision "C" in an open state to facilitate access to the surgical site. Moreover, the seal anchor member 100 is configured and adapted to inhibit the flow of gasses to and from the surgical site, while facilitating the insertion of surgical instruments "I" (e.g., cannulae) into the surgical site.

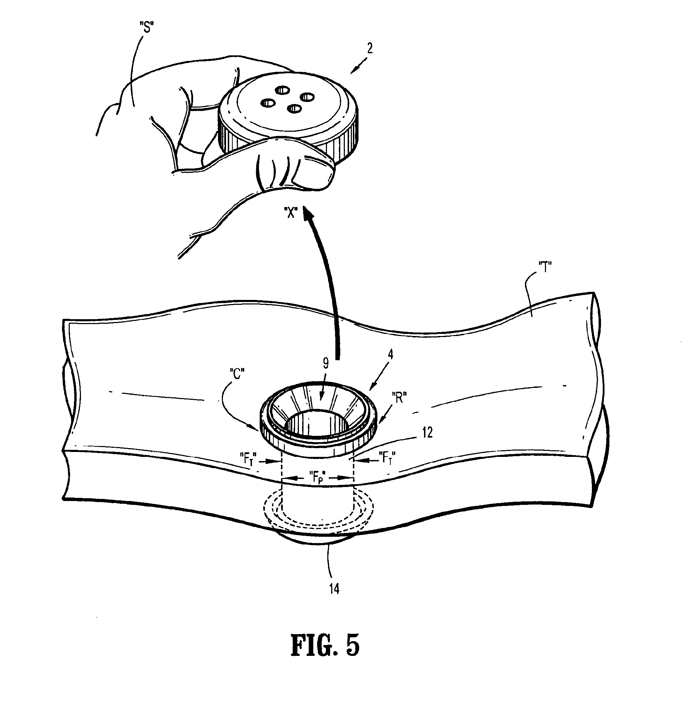

The seal anchor member 100 includes a body portion 4 and a cap 2. The seal anchor member 100 is adapted to be reconfigured between a first state (Figs. 1-4) in which the cap 2 is releasably secured to the body portion 4, and a second state in which the cap 2 is separated from the body portion 4 (Fig. 5). The body portion 4 includes a central lumen 9 that is disposed along longitudinal axis "A", and the cap 2 includes one or more openings 8 that each defines a diameter that is smaller than the diameter of the central lumen 9. Advantageously, this allows a surgeon "S" (Fig. 5) to use a single seal anchor member 100 throughout the course of a minimally invasive procedure, where access requirements to the surgical site might have necessitated removal of a similarly sized seal anchor member that is not reconfigurable between first and second states.

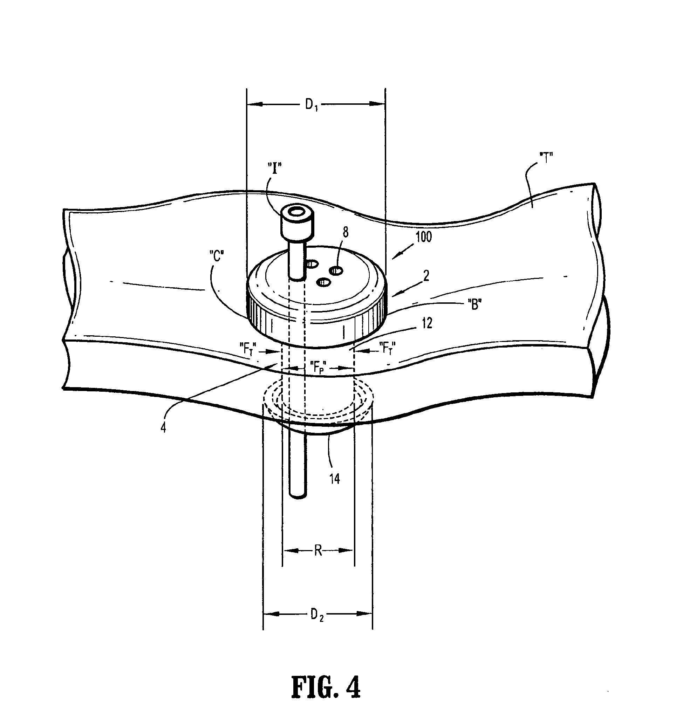

In the first state, the seal anchor member 100 includes respective trailing and leading ends 13, 14. The trailing and leading ends 13, 14 may define surfaces that are substantially arcuate to assist in the insertion of the seal anchor member 100 within a tissue tract "B" of the incision "C" of the tissue "T". In addition, the body portion 4 includes an intermediate portion 12 that includes a radial dimension "R" (Fig. 4). The radial dimension "R" may vary along the longitudinal axis "A", such that the seal anchor member 100 defines a cross-sectional dimension that varies along the longitudinal axis "A", which facilitates anchoring of the seal anchor member 100 within the tissue "T". Moreover, the radial dimension "R" of the intermediate portion 12 may be appreciably less than the respective diameters D1, D2 of the trailing and leading ends of the seal anchor member 100, such that the seal anchor member 100 defines an "hour-glass" shape or configuration. However, embodiments in which the radial dimension "R" of the seal anchor member 100 remains substantially uniform is also within the scope of the present disclosure. In addition, the diameters D1, D2 of the trailing and leading ends of the seal anchor member 100 may be the same or different. In cross-section, the seal anchor member 100 may exhibit any suitable configuration, e.g., substantially circular, oval, or oblong.

As discussed above, the cap 2 includes one or more openings 8. Each opening 8 may be disposed along the longitudinal axis "A" and is configured and adapted to removably receive a surgical instrument "I" in a substantially sealed relation. In particular, each opening 8 has a slightly smaller diameter than instrument "I" such that it transitions (i.e., expands slightly) to approximate the diameter of the surgical instrument "I" inserted therein such that the flow of gasses through opening 8 is inhibited in the presence of the surgical instrument "I" inserted therein (i.e., forms a substantially fluid-tight barrier). In addition, the one or more openings 8 may each have the same or different diameters. Moreover, the openings 8 may be equidistantly spaced or some openings 8 may be closer to one another than other of the openings 8. Although four (4) openings 8 are shown, it is within the scope of the present disclosure that a fewer or greater number of openings may be provided in cap 2.

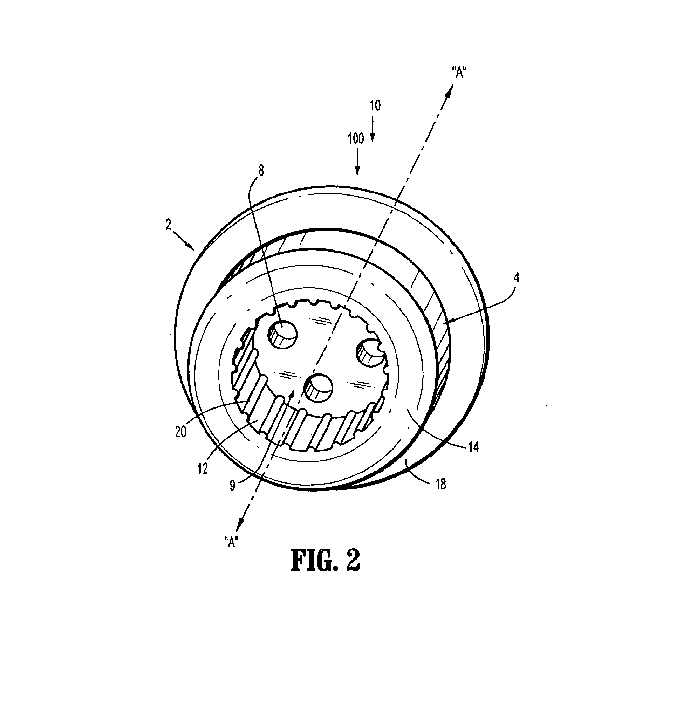

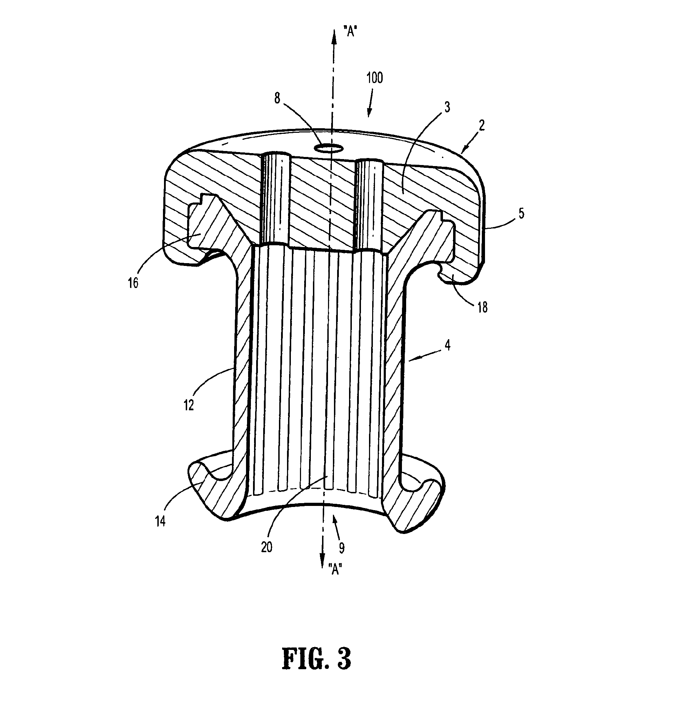

As seen in Figs. 2 and 3, the body portion 4 includes a central lumen 9 disposed along the longitudinal axis "A". Ribbing 20 along the surface of the intermediate portion 12 of body portion 4 may facilitate maintenance of the structural rigidity of the body portion 4 to resist compression when placed within an incision "C" of tissue "T" (Figs. 4 and 5). The cap 2 includes one or more openings 8 that each defines a diameter that is less than the diameter of the lumen 9 of the body portion.

The seal anchor member 100 may be formed from a compressible or semi-rigid material having an internal biasing force, such that the material may be deformed and will transition toward an initial configuration after being deformed. As shown in Fig. 4, the internal biasing force "Fp" of the body portion 4 will resist the compressive forces "Ft" of the incision "I". As shown in Figs. 4 and 5, the seal anchor member 100 may be placed within an incision "C" of a tissue "T". Each opening 8 is adapted and configured to receive a surgical instrument "I" therein in a substantially sealed relation.

Advantageously, as shown in Fig. 5, a surgeon "S" may remove the cap 2 by applying a force in the direction of arrow "X" while leaving the body portion 4 within the incision "C" of the tissue "T", thereby reconfiguring the seal anchor member 100 to the second state. Greater access to the surgical site is facilitated by the removal of cap 2 and providing unrestricted access to the central lumen 9. Central lumen 9 is dimensioned to accommodate larger surgical instruments "I" (e.g., specimen retrieval devices) than would be accommodated by openings 8. Moreover, in some embodiments the lumen 9 may facilitate reception of a hand of the surgeon "S" and the digital manipulation of body structures within the surgical site by the surgeon "S".

Moreover, with the body portion 4, remaining in place within incision "C" and tissue tract "B", the internal biasing force "Fp" of body portion 4 resists the compressive forces "Ft" of the tissue tract "B" of incision "C", thereby facilitating access to the surgical site. In addition, depending upon the particular application, a different cap, including differently configured openings, may be removably secured to the body portion 4.

Seal anchor member 100 may be formed from a suitable foam material having sufficient compliance to form a seal about one or more surgical objects, shown generally as surgical object "I" (FIG. 4), and also establish a sealing relation with the tissue. The foam is preferably sufficiently compliant to accommodate off axis motion of the surgical object "I". In one embodiment, the foam includes a polyisoprene material. For example, the seal anchor member 100 is formed of a flowable or sufficiently compliable material such as a foam material, e.g., an open-cell polyurethane foam, a thermoplastic elastomer (TPE) or a gel. In other embodiments, the seal anchor member 100 may be formed from a rubber or rubber-like material. Moreover, the seal anchor member 100 may be formed from a semi-rigid material such that the intermediate portion 12 may resist deformation when placed within the incision "C" and tissue tract "B" of the patient's tissue "T".

Although the presently disclosed seal anchor member 100 is shown with an incision through tissue, it is contemplated that the seal anchor member may be used in any procedure where access is through a naturally occurring orifice (e.g. vagina or anus).

Although the illustrative embodiments of the present disclosure have been described herein with reference to the accompanying drawings, the above description, disclosure, and figures should not be construed as limiting, but merely as exemplifications of particular embodiments. It is to be understood, therefore, that the disclosure is not limited to those precise embodiments, and that various other changes and modifications may be effected therein by one skilled in the art without departing from the scope or spirit of the disclosure.

The invention may be described by reference to the following numbered paragraphs:
1. A surgical apparatus for positioning within a tissue tract accessing an underlying body cavity, which comprises:
   a seal anchor member having a longitudinal axis, the seal anchor member including a compressible body portion having an inner surface defining a lumen along a longitudinal axis of the seal anchor member, the lumen defining a first diameter and having ribbing along the inner surface.
2. The surgical apparatus of paragraph 1, wherein the ribbing increases the structural rigidity of the flexible body portion.
3. The surgical apparatus of paragraph 1, wherein the ribbing resists compression when placed within the incision.
4. The surgical apparatus of paragraph 1, wherein the ribbing extends longitudinally along the inner surface.
5. The surgical apparatus of paragraph 4, wherein the ribbing extends from the proximal end of the compressible body portion to the distal end of the compressible body portion.
6. The surgical apparatus of paragraph 1, further comprising a cap that is releasably securable to a proximal end of the seal anchor member.
7. The surgical apparatus of paragraph 6, wherein the cap includes one or more openings extending along the longitudinal axis, each opening defining a diameter, each diameter being less than the first diameter of the lumen of the seal anchor member.
8. The surgical apparatus of paragraph 6, wherein each opening of the cap is sized to receive a surgical object.
9. A surgical apparatus for positioning within a tissue tract accessing an underlying body cavity, which comprises a body portion having an inner surface defining a lumen along a longitudinal axis of the seal anchor member, the body portion being selectively compressible so as to be inserted within a tissue tract, the lumen having ribbing along the inner surface, the ribbing increasing the structural rigidity of the flexible body portion such that, after the body portion has already been positioned within the incision, the ribbing helps maintain the lumen open by resisting compression forces exerted on the body portion by the tissue tract.
10. The surgical apparatus of paragraph 9, wherein the ribbing extends longitudinally along the inner surface.
11. The surgical apparatus of paragraph 10, wherein the ribbing extends from the proximal end of the compressible body portion to the distal end of the compressible body portion.

## Claims

1. A surgical apparatus for positioning within a tissue tract accessing an underlying body cavity, which comprises:
a seal anchor member having a longitudinal axis, the seal anchor member including a compressible body portion having an inner surface defining a lumen along a longitudinal axis of the seal anchor member, the lumen defining a first diameter and having ribbing along the inner surface.

2. The surgical apparatus of claim 1, wherein the ribbing increases the structural rigidity of the flexible body portion.

3. The surgical apparatus of claim 1 or claim 2, wherein the ribbing resists compression when placed within the incision.

4. The surgical apparatus of any preceding claim, wherein the ribbing extends longitudinally along the inner surface.

5. The surgical apparatus of claim 4, wherein the ribbing extends from the proximal end of the compressible body portion to the distal end of the compressible body portion.

6. The surgical apparatus of any preceding claim, further comprising a cap that is releasably securable to a proximal end of the seal anchor member.

7. The surgical apparatus of claim 6, wherein the cap includes one or more openings extending along the longitudinal axis, each opening defining a diameter, each diameter being less than the first diameter of the lumen of the seal anchor member.

8. The surgical apparatus of claim 7, wherein each opening of the cap is sized to receive a surgical object.

9. A surgical apparatus for positioning within a tissue tract accessing an underlying body cavity, which comprises a body portion having an inner surface defining a lumen along a longitudinal axis of the seal anchor member, the body portion being selectively compressible so as to be inserted within a tissue tract, the lumen having ribbing along the inner surface, the ribbing increasing the structural rigidity of the flexible body portion such that, after the body portion has already been positioned within the incision, the ribbing helps maintain the lumen open by resisting compression forces exerted on the body portion by the tissue tract.

10. The surgical apparatus of claim 9, wherein the ribbing extends longitudinally along the inner surface.

11. The surgical apparatus of claim 10, wherein the ribbing extends from the proximal end of the compressible body portion to the distal end of the compressible body portion.
